# EUROPEAN PATENT APPLICATION

(11) **EP 2 894 582 A1**
(43) Date of publication of application: **15.07.2015**
(21) Application number: 14185624.5
(22) Date of filing: 19.09.2014
(51) Int. Cl.: G06F 19/00

(54) **Method, system and apparatus for calculating the insulin-to-carbohydrate ratio for diabetics**

(30) Priority: 09.01.2014 US 201414151392
(71) Applicant: Thomson, Caren Frances, Delta, British Columbia V4E 2V8 (CA)
(72) Inventor: Thomson, Caren Frances, Delta, British Columbia V4E 2V8 (CA)
(74) Representative: Newman, Alastair James Morley

(57) **Abstract**

A more accurate method of determining an Insulin to Carbohydrate Ratio for an individual diabetic is provided. The individual, who has been using or has been given a prior suggested dosage of rapid-acting insulin to be taken prior to meals, first plans a benchmark meal ("the Meal") and precisely counts the number of grams of carbohydrates which will be consumed in the meal. The individual blood glucose level is measured before the meal, then the previous dosage of rapid-acting insulin is injected. After the meal another reading is taken of the individual's blood sugar level. The number of units X of carbohydrates for each unit of rapid-acting insulin to be taken before future meal is then calculated by a unique formula.

## Description

### Cross Reference To Related Application

The present application is a continuation-in-part of United States patent application no. 14/122,749 filed November 27, 2013, which claims the benefits, under 35 U.S.C.§119(e), of U.S. Provisional Application Serial No. 61/670,275 filed July 11, 2012 both of which are incorporated herein by this reference.

### Technical Field

The invention relates to the field of diabetes management and methods and systems for diabetic individuals to set, or have set for them, their individual insulin dosages.

### Background

An increasing proportion of the population is affected by diabetes, either Type I where the individual's pancreas has stopped producing insulin due to destruction of the insulin-producing islet cells, or Type II where the individual's pancreas produces insufficient insulin resulting in insulin deficiency, and/or the individual's cells are resistive to insulin resulting in insulin resistance. In all cases of Type I diabetes, and many cases of Type II diabetes, regular and ongoing injections of insulin are necessary to avoid hyperglycemia. Since multiple injections per day may be necessary, most diabetics self-inject their own insulin. Currently this is generally done with an insulin pen, although increasing numbers are using insulin infusion pumps such as the Medtronic MiniMed^{tm}. Individuals monitor their own blood sugar level to ensure it is within a safe range, using blood glucose (BG) monitors.

It is important for the effectiveness of the insulin injection regime, in order to avoid hyperglycemia or hypoglycemia, that the proper dosage of insulin be given at each injection depending on the meals to be consumed by the individual, as well as other factors such as exercise, stress, female hormonal changes etc. Generally the best results are achieved by administering two types of insulin: i) basal insulin which acts over a 24 hour period, normally injected in the evening, to provide a baseline foundation of insulin, and ii) bolus or rapid-acting insulin, such as NovoRapid^{tm}, Humalog^{tm} or Apidra^{tm}, which is injected preferably before or after meals to address the carbohydrate intake associated with meals.

The individual must estimate the amount of bolus insulin to be administered before meals depending on the amount of carbohydrates to be consumed in the meal. For this purpose the individual diabetic must count the number of grams of carbohydrates to be consumed for each meal and then determine the number of units of bolus insulin to administer prior to the meal based on the individual's Insulin to Carbohydrate Ratio (ICR). Conventionally, calculating the ICR was done by applying certain conventional rules and then adjusting by trial and error. The conventional rule, called the "500 Rule", states that the individual should divide 500 by the number of units of both basal and bolus insulin taken by the individual per day, to provide the ICR.

It is well known that the various conventional rules can be very inaccurate depending on the multitude of individual factors which affect a correct ICR ratio for a given individual. There is therefore a need for a more accurate method of determining an accurate ICR for an individual diabetic.

The foregoing examples of the related art and limitations related thereto are intended to be illustrative and not exclusive. Other limitations of the related art will become apparent to those of skill in the art upon a reading of the specification and a study of the drawings.

### Summary

The following embodiments and aspects thereof are described and illustrated in conjunction with systems, tools and methods which are meant to be exemplary and illustrative, not limiting in scope. In various embodiments, one or more of the above-described problems have been reduced or eliminated, while other embodiments are directed to other improvements.

The present invention therefore provides a more accurate method of determining an accurate ICR for an individual diabetic. The individual, who has been using or has been given a prior suggested dosage of rapid-acting insulin to be taken prior to meals, first plans a benchmark meal ("the Meal") and precisely counts the number of grams of carbohydrates which will be consumed in the Meal. Using a blood glucose monitor or meter, the individual measures his/her blood glucose level before the meal BG₁, then injects his/her previous dosage of rapid-acting insulin using an insulin pen or insulin infusion pump. After the meal another reading is taken of the individual's blood sugar level. The number of units X of carbohydrates for each unit of Rapid Insulin to be taken in a meal is then calculated as follows:
i. Calculate A = (BG₁ - 7)/ I, where BG₁ is the blood sugar value in mmol/L, within one-half hour prior to the Meal, which must be greater than or equal to 4 mmol/L. If BG₁ 7, then BG₁ is set as 7. I is the Insulin Sensitivity Factor as defined below.
ii. Calculate B = (BG₂ - 10)/ I, where BG₂ is the blood sugar value in mmol, one and one-half to two hours from the start of the Meal, which must be greater than or equal to 5 mmol/L. If BG₂ 10, then BG₂ is set as 10.
iii. C = B-A
iv. E = C + D, where D is the number of units of Rapid Insulin taken prior to the Meal.
v. X = Y/E, where Y is the number of grams of carbohydrates consumed in the Meal.
vi. The individual will then enter the number Z/X as the number of units of rapid-acting insulin to inject prior to a meal at which Z grams of carbohydrates will be consumed, modified by any corrective factors.

Preferably the calculation is made by the individual entering the results of the two blood sugar readings and the number of carbohydrates in the Meal into an application on a mobile hand-held device or a personal computer. The invention therefore also provides a computer programmed to carry out the foregoing method, and a system for implementing the method comprising a blood glucose monitor, a computer for carrying out the calculation and a device for injecting insulin, for use by a diabetic individual. The result of the calculation (ICR) can also be stored directly in an insulin pump and used to calculate ongoing insulin dosages.

In addition to the exemplary aspects and embodiments described above, further aspects and embodiments will become apparent by reference to the drawings and by study of the following detailed descriptions.

### Brief Description of Drawings

Exemplary embodiments are illustrated in referenced figures of the drawings. It is intended that the embodiments and figures disclosed herein are to be considered illustrative rather than restrictive.

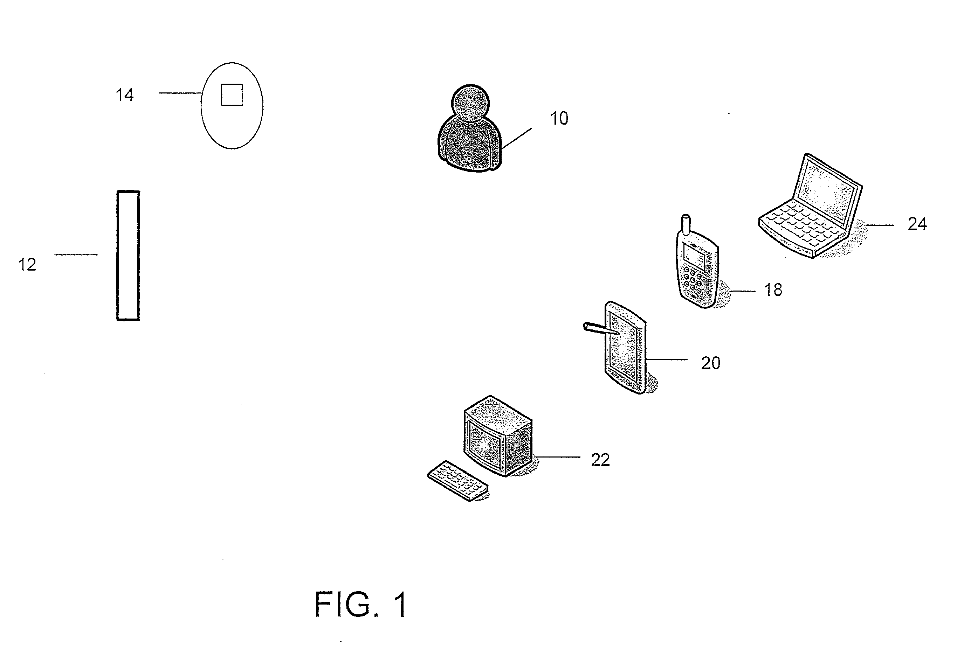

Figure 1 is a schematic diagram of a system according to a first embodiment of the invention;

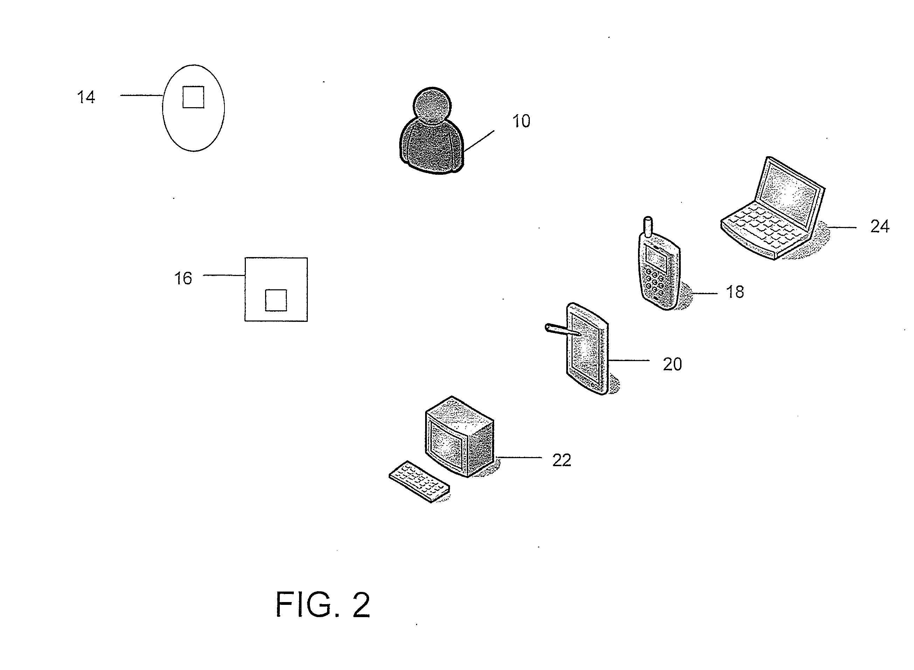

Figure 2 is a schematic diagram of a system according to a first embodiment of the invention;

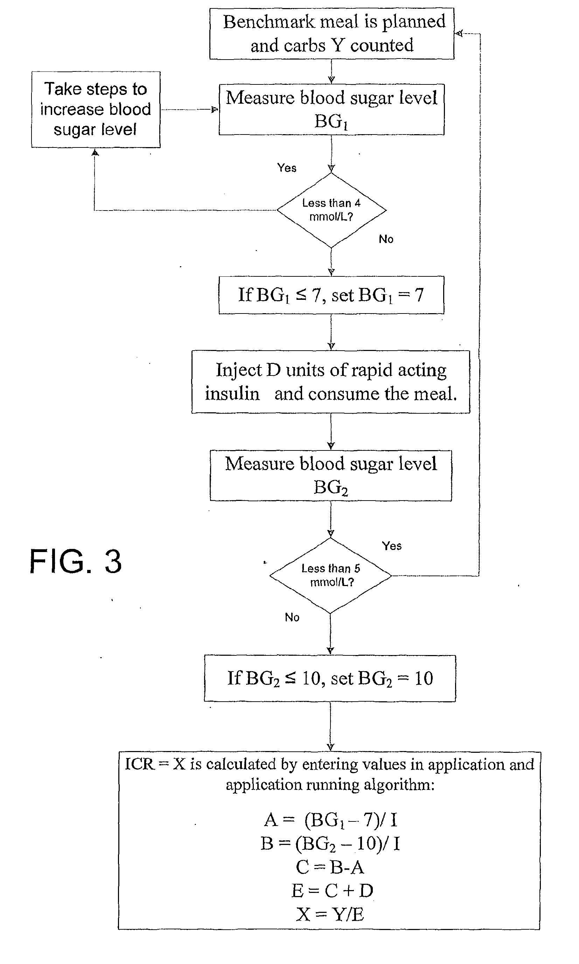

Figure 3 is a flowchart illustrating the method of the invention.

### Description

Throughout the following description specific details are set forth in order to provide a more thorough understanding to persons skilled in the art. However, well known elements may not have been shown or described in detail to avoid unnecessarily obscuring the disclosure. Accordingly, the description and drawings are to be regarded in an illustrative, rather than a restrictive, sense.

An individual diabetic 10, who requires injections of insulin, will typically self-inject the insulin multiple times per day using an insulin pen 12, such as a NovoPen manufactured by Novo Nordisk, or an insulin infusion pump 16 such as the Medtronic MiniMed^{tm}, Animas or Omnipod^{tm} and will periodically measure his or her blood sugar level using a blood glucose (BG) monitor 14, such as manufactured by LifeScan Inc., Abbott Diabetes Care Inc. and others. That individual will be taking a single daily injection of basal insulin and will initially have a set number D of units of bolus/ rapid-acting insulin for taking prior to each meal, calculated according to one of the conventional rules. In order to establish a relatively accurate Insulin to Carbohydrate Ratio (ICR) for himself or herself for future injections of the rapid-acting, bolus insulin, the individual 10 follows the following method.

First, a benchmark meal is planned ("the Meal"). The individual must carefully count the number of grams of carbohydrates Y to be consumed in the Meal using accepted carb counting methods. Within approximately one-half hour prior to the Meal, the individual 10 measures his/her blood sugar level BG₁ using a blood glucose (BG) monitor 14. That reading must be greater than or equal to 4 mmol/L (millimoles per liter). If it is less than 4 mmol/L, the individual must consume some sugar, for example orange juice, to bring it over 4 mmol/L. If it is greater than or equal to 4 mmol/L, the individual injects the D units of rapid-acting insulin. The individual then consumes the Meal. Starting the clock with the first bite of the Meal, one and one-half to two hours after the start of the Meal, the individual measures his/her blood sugar level BG₂ using a blood glucose (BG) monitor 14. That second reading must be greater than or equal to 5 mmol/L. If it is less than 5 mmol/L, the individual took too much rapid- acting insulin before the Meal and must start over again. If it is greater than or equal to 5 mmol/L, the individual can then carry out the calculation to determine the individual's Insulin to Carbohydrate Ratio (ICR) = X.

The following calculation is then made to determine the individual's ICR based on the foregoing readings, where for an ICR = X, 1 unit of rapid Insulin is required to be injected for X grams of carbohydrate consumed in the subject meal. Preferably the calculation is made by an application downloaded onto a hand-held mobile device such as an iPod Touch, iPhone, Android, iPad or a personal computer:
i. Calculate A = (BG₁ - 7)/ I, where BG₁ is the blood sugar value in mmol/L, (measured within about one-half hour prior to the Meal), which must be greater than or equal to 4 mmol/L. If BG₁ 7, then BG₁ is set as 7. I is the Insulin Sensitivity Factor as defined below.
ii. Calculate B = (BG₂ - 10)/ I, where BG₂ is the blood sugar value in mmol/L, (measured about one and one-half to two hours from the start of the Meal), which must be greater than or equal to 5 mmol/L. If BG₂ 10, then BG₂ is set as 10.
iii. C = B-A
iv. E = C + D, where D is the number of units of rapid-acting insulin taken prior to the Meal.
v. X = Y/E, where Y is the number of grams of carbohydrates consumed in the Meal.

The "Insulin Sensitvity Factor" (ISF) or "Correction Factor" (CF) is defined as the amount (in mmol/L) by which 1 unit of Rapid (mealtime) insulin lowers an individual's blood sugar. For example, using measurement units in mmol/L, if 1 unit of Rapid (mealtime) insulin lowers the individual's blood sugars by 2 mmol/L, the ISF (Insulin Sensitivity Factor) or CF (Correction Factor) is 2. The ISF can vary in some individuals but Type 2 adult diabetics typically have an ISF or CF of 2 and Type 1 adult diabetics typically have an ISF or CF of from about 3 to 4.

To utilize the ICR, prior to a future meal, the individual will calculate the number of grams of carbohydrates Z to be eaten in the meal and then enter on his/her insulin pen the number (Z/X) as the number of units of rapid-acting insulin to inject prior to the meal, modified by any corrective factors required to be taken into consideration for any given meal. Normally the calculated ratio X will apply for all meals of the individual, but certain individuals may be required to modify the ratio for breakfast. A corrective factor will probably be required if the individual has had increased exercise or is planning increased exercise within 2 hours before or after a meal. Other corrective factors include female hormonal changes, infection, increased stress, steroid therapy, chemotherapy or some antidepressant or some blood pressure medications. Where corrective factors are involved, it will be necessary for the individual to take blood sugar readings before and after the meal and calculate the new ICR.

The foregoing method requires that the blood sugar reading be taken in mmol/L. Since 18 mg/dL = 1 mmol/L, if the blood sugar reading is taken in mg/dL it must be divided by 18. A variation of roughly plus or minus 20% can be made from the measurements, times and factors in the above measurements and calculations without affecting the usefulness of the resultant ratio.

The algorithm described above is preferably programmed into an application downloaded onto an information processing device such as a hand-held mobile device 18 such as an iPhone, a tablet 20 such as an iPad, a laptop 24 or a personal desktop computer 22. Such an information processing device has data entry such as a keyboard or touch screen, data storage, data processor and data output, such as a display screen or printer. To carry out the method, the individual carries out the required calculation of Y, measurements of BG₁ and BG₂, enters the values via the keyboard of the information processing device, runs the application and stores the ICR value X for future use. The application may also be stored in the memory of an insulin infusion pump and run directly on the insulin pump, and the calculation stored in the memory of the insulin pump to be used in calculating ongoing insulin dosages.

While in the preferred embodiment the individual diabetic 10 himself or herself makes the calculation of Y, injects the insulin amount D, measures BG₁ and BG₂ and enters the values in an information processing device, the individual 10 can be assisted in one or more of these steps by one or more other individuals, for example if the individual 10 is disabled or too young or old to be able to take such steps.

While a number of exemplary aspects and embodiments have been discussed above, those of skill in the art will recognize certain modifications, permutations, additions and sub-combinations thereof. It is therefore intended that the following appended claims and claims hereafter introduced are interpreted to include all such modifications, permutations, additions and sub-combinations as are within their true spirit and scope.

## Claims

1. A method of setting a dosage of rapid-acting insulin to be administered before a given meal to an individual diabetic by estimating Insulin to Carbohydrate Ratio for the individual diabetic, wherein a dosage of rapid-acting insulin has been set for the individual diabetic to take prior to meals, said method comprising:
i) planning a benchmark meal and counting the number of grams of carbohydrates Y which will be consumed in the benchmark meal;
ii) measuring the blood glucose level BG₁ of the individual within a pre-defined period prior to the benchmark meal;
iii) administering the set dosage D units of rapid-acting insulin;
iv) taking a second reading of the individual's blood sugar level BG₂ after the benchmark meal has been consumed within a pre-defined period after the start of the benchmark meal;
v) calculating the Insulin to Carbohydrate Ratio X for rapid-acting insulin, where X is the number of grams of carbohydrates in a meal per unit of rapid-acting insulin to be taken prior to the meal, as follows:
a) calculating A = (BG₁ 7)/ I, where BG₁ is the blood sugar value in mmol/L, which must be greater than or equal to 4 mmol/L; if BG₁ ≤ 7, then set BG₁ as 7; and where I is the Insulin Sensitivity Factor;
b) calculating B = (BG₂ - 10)/ I, where BG₂ is the blood sugar value in mmol/L, which must be greater than or equal to 5 mmol/L; if BG₂ ≤ 10, then set BG₂ as 10;.
c) calculating X = Y/E, where E = (B-A) + D;
d) for said given meal, counting the number Z grams of carbohydrates to be consumed and determining Z/X as the number of units of rapid-acting insulin to inject prior the given meal.

2. The method of claim 1 wherein the calculated Insulin to Carbohydrate Ratio X is modified for the given meal by a corrective factor selected from the group consisting of whether the given meal is breakfast, increased exercise before or after the given meal, female hormonal changes, infection, increased stress, steroid therapy, chemotherapy, use of anti-depressants, use of blood pressure medications and combinations of the foregoing factors.

3. The method of claim 1 wherein the pre-defined period prior to the benchmark meal is less than approximately one-half hour.

4. The method of claim 1 wherein the pre-defined period after the start of the benchmark meal is approximately one and one-half to two hours.

5. The method of claim 1 wherein the blood glucose levels are measured using a blood glucose monitor or meter.

6. The method of claim 1 wherein I is approximately 2.

7. The method of claim 1 wherein I is between approximately 3 and 4.

8. Rapid-acting insulin for use in a method of setting a dosage of rapid-acting insulin to be administered before a given meal to an individual diabetic by estimating Insulin to Carbohydrate Ratio for the individual diabetic, wherein a dosage of rapid-acting insulin has been set for the individual diabetic to take prior to meals, said method comprising:
i) planning a benchmark meal and counting the number of grams of carbohydrates Y which will be consumed in the benchmark meal;
ii) measuring the blood glucose level BG₁ of the individual within a pre-defined period prior to the benchmark meal;
iii) administering the set dosage D units of rapid-acting insulin;
iv) taking a second reading of the individual's blood sugar level BG₂ after the benchmark meal has been consumed within a pre-defined period after the start of the benchmark meal;
v) calculating the Insulin to Carbohydrate Ratio X for rapid-acting insulin, where X is the number of grams of carbohydrates in a meal per unit of rapid-acting insulin to be taken prior to the meal, as follows:
a) calculating A = (BG₁ - 7)/ I, where BG₁ is the blood sugar value in mmol/L, which must be greater than or equal to 4 mmol/L; if BG, ≤ 7, then set BG₁ as 7; and where I is the Insulin Sensitivity Factor;
b) calculating B = (BG₂ - 10)/I, where BG₂ is the blood sugar value in mmol/L, which must be greater than or equal to 5 mmol/L; if BG₂ ≤ 10, then set BG₂ as 10;
c) calculating X = Y/E, where E = (B-A) + D;
d) for said given meal, counting the number Z grams of carbohydrates to be consumed and determining Z/X as the number of units of rapid-acting insulin to inject prior the given meal.

9. Rapid-acting insulin for use in treating a diabetic patient by administration of an amount (Z/X) of rapid-acting insulin before a given meal, the amount of rapid-acting insulin administered being determined by estimating Insulin to Carbohydrate ratio for the diabetic patient in a method comprising:
i) planning a benchmark meal and counting the number of grams of carbohydrates Y which will be consumed in the benchmark meal;
ii) measuring the blood glucose level BG₁ of the diabetic patient within a pre-defined period prior to the benchmark meal;
iii) administering a set dosage D units of rapid-acting insulin;
iv) taking a second reading of the diabetic patient's blood sugar level BG₂ after the benchmark meal has been consumed within a pre-defined period after the start of the benchmark meal;
v) calculating the Insulin to Carbohydrate Ratio X for rapid-acting insulin, where X is the number of grams of carbohydrates in a meal per unit of rapid-acting insulin to be taken prior to the meal, as follows:
a) calculating A = (BG₁ - 7)/ I, where BG₁ is the blood sugar value in mmol/L, which must be greater than or equal to 4 mmol/L; if BG₁ ≤ 7, then set BG₁ as 7; and where I is the Insulin Sensitivity Factor;
b) calculating B = (BG₂ - 10)/I, where BG₂ is the blood sugar value in mmol/L, which must be greater than or equal to 5 mmol/L; if BG₂ ≤ 10, then set BG₂ as 10;
c) calculating X = Y/E, where E = (B-A) + D;
d) for said given meal, counting the number Z grams of carbohydrates to be consumed and determining Z/X as the number of units of rapid-acting insulin to inject prior the given meal.

10. A system for setting a dosage of rapid-acting insulin to be administered before a given meal to an individual diabetic by estimating an Insulin to Carbohydrate Ratio for the individual diabetic, wherein a dosage of rapid-acting insulin has been set for the individual diabetic to take prior to meals, said system comprising:
i) a blood glucose measuring apparatus;
ii) an insulin injection device; and
iii) an information processing device having stored thereon program code which, when executed by the information processing device, performs the following calculation for setting a dosage of rapid-acting insulin to be administered before a given meal to an individual diabetic, where a benchmark meal has been planned at which Y grams of carbohydrates are consumed, where the blood glucose level BG₁ of the individual was measured within a pre-defined period prior to the benchmark meal, where D units of rapid-acting insulin were administered prior to the benchmark meal, and where a second reading of the individual's blood sugar level BG₂ was measured after the benchmark meal was consumed within a pre-defined period after the start of the benchmark meal said calculation comprising:
calculating the Insulin to Carbohydrate Ratio X for rapid-acting insulin, where X is the number of grams of carbohydrates in a meal per unit of rapid-acting insulin to be taken prior to the meal, as follows:
a) calculating A = (BG₁ - 7)/ I, where BG₁ is the blood sugar value in mmol/L, which must be greater than or equal to 4 mmol/L; if BG₁ ≤ 7, then set BG₁ as 7; and where I is the Insulin Sensitivity Factor;
b) calculating B = (BG₂ - 10)/I, where BG₂ is the blood sugar value in mmol/L, which must be greater than or equal to 5 mmol/L; if BG₂ ≤ 10, then set BG₂ as 10;
c) calculating X = Y/E, where E = (B-A) + D;
d) for said given meal where Z grams of carbohydrates is to be consumed, determining Z/X as the number of units of rapid-acting insulin to inject prior the given meal.

11. The system of claim 10 wherein
- the calculated Insulin to Carbohydrate Ratio X is modified for the given meal by a corrective factor selected from the group consisting of whether the given meal is breakfast, increased exercise before or after the given meal, female hormonal changes, infection, increased stress, steroid therapy, chemotherapy, use of anti-depressants, use of blood pressure medications and combinations of the foregoing factors; or
- the pre-defined period prior to the benchmark meal is less than approximately one-half hour; or
- the pre-defined period after the start of the benchmark meal is approximately one and one-half to two hours.

12. The system of claim 10 wherein I is approximately 2.

13. The system of claim 10 wherein I is between approximately 3 and 4.

14. An information processing device, comprising a data entry, data storage, data processor and data output, said data storage having program code stored therein, wherein the program code, when executed by a computer, performs the following calculation for setting a dosage of rapid-acting insulin to be administered before a given meal to an individual diabetic, where a benchmark meal has been planned at which Y grams of carbohydrates are consumed, where the blood glucose level BG₁ of the individual was measured within a pre-defined period prior to the benchmark meal, where D units of rapid-acting insulin were administered prior to the benchmark meal, and where a second reading of the individual's blood sugar level BG₂ was measured after the benchmark meal was consumed within a pre-defined period after the start of the benchmark meal, said calculation comprising:
i) calculating the Insulin to Carbohydrate Ratio X for rapid-acting insulin, where X is the number of grams of carbohydrates in a meal per unit of rapid-acting insulin to be taken prior to the meal, as follows:
a) calculating A = (BG₁ - 7)/ I, where BG₁ is the blood sugar value in mmol/L, which must be greater than or equal to 4 mmol/L; if BG₁ ≤ 7, then set BG₁ as 7; and where I is the Insulin Sensitivity Factor;
b) calculating B = (BG₂ - 10)/I, where BG₂ is the blood sugar value in mmol/L, which must be greater than or equal to 5 mmol/L; if BG₂ ≤ 10, then set BG₂ as 10;
c) calculating X = Y/E, where E = (B-A) + D;
d) for said given meal, where Z grams of carbohydrates is to be consumed, determining Z/X as the number of units of rapid-acting insulin to inject prior the given meal.

15. A computer readable storage medium having program code stored thereon, wherein the program code, when executed by a computer, determines a dosage of rapid-acting insulin to be administered before a given meal to an individual diabetic, where a benchmark meal has been planned at which Y grams of carbohydrates are consumed, where the blood glucose level BG₁ of the individual was measured within a pre-defined period prior to the benchmark meal, where D units of rapid-acting insulin were administered prior to the benchmark meal, and where a second reading of the individual's blood sugar level BG₂ was measured after the benchmark meal was consumed within a pre-defined period after the start of the benchmark meal, said determination comprising:
i) calculating the Insulin to Carbohydrate Ratio X for rapid-acting insulin, where X is the number of grams of carbohydrates in a meal per unit of rapid-acting insulin to be taken prior to the meal, as follows:
a) calculating A = (BG₁ - 7)/ I, where BG₁ is the blood sugar value in mmol/L, which must be greater than or equal to 4 mmol/L; if BG₁ ≤ 7, then set BG₁ as 7; and where I is the Insulin Sensitivity Factor;
b) calculating B = (BG₂ - 10)/I, where BG₂ is the blood sugar value in mmol/L, which must be greater than or equal to 5 mmol/L; if BG₂ ≤ 10, then set BG₂ as 10;
c) calculating X = Y/E, where E = (B-A) + D;
d) for said given meal, where Z grams of carbohydrates is to be consumed, determining Z/X as the number of units of rapid-acting insulin to inject prior the given meal.
